# DEMANDE DE BREVET EUROPEEN

(11) **EP 2 786 736 A1**
(43) Date de publication de la demande: **08.10.2014**
(21) Numéro de dépôt: 14169287.1
(22) Date de dépôt: 17.10.2001
(51) Int. Cl.: A61K 8/06, A61Q 13/00

(54) **Procédé de fabrication d'une composition parfumante sans alcool**

(30) Priorité: 20.10.2000 CH 20602000
(62) Demande divisionnaire de: 01974619.7
(71) Demandeur: Firmenich SA, 1211 Geneva 8 (CH)
(72) Inventeur: Stora, Thierry, 01710 Thoiry (FR); Daugeron, Aude, 92200 Neuilly-sur-Seine (FR); Mounier, Rémy, 92200 Neuilly-sur-Seine (FR); Personnic, Nathalie, 92200 Neuilly-sur-Seine (FR)
(74) Mandataire: Baumgartner Harris, Pauline

(57) **Abrégé**

Procédé de fabrication d'une composition parfumante sans alcool sous forme d'une émulsion vaporisable huile-dans-l'eau translucide comprenant les étape suivante :
a) mélanger un système de tensioactif rendu liquide et la phase huileuse constitué essentiellement d'ingrédients parfumants à température ambiante jusqu'à l'obtention d'un mélange homogène ;
b) ajouter au mélange obtenu à l'étape a) une phase aqueuse ;
c) chauffer le mélange obtenu à l'étape b) tout en maintenant une faible agitation pour former une émulsion de viscosité inférieure à 1 Pa.s. ;
d) stopper le chauffage et refroidir à température ambiante sous agitation.

## Description

### Domaine Technique

La présente invention a trait au domaine de la parfumerie. Elle concerne plus particulièrement une composition parfumante concentrée sans alcool, sous forme d'une émulsion huile-dans-l'eau translucide. Ladite composition parfumante peut être utilisée notamment sous forme d'émulsion parfumée susceptible d'être vaporisée sur la peau ou les cheveux, sur tout autre type de surface ou encore simplement dans l'air ambiant.

### Technique Antérieure

Les émulsions huile-dans-l'eau sont bien connues dans le domaine de la cosmétique et de la dermatologie, notamment pour la préparation de produits cosmétiques tels que des crèmes, lotions, toniques ou sérums. De telles émulsions sont par exemple décrites dans le brevet européen EP 728 460 qui concerne plus particulièrement des nanoémulsions transparentes à base de lipides amphiphiles non-ioniques fluides et leur utilisation en cosmétique et en dermopharmacie. Ces émulsions contiennent toujours une faible proportion d'un alcool de faible poids moléculaire. Par ailleurs, les problèmes liés à la formulation d'émulsions dans le domaine de la cosmétique ne sont pas les mêmes qu'en parfumerie. En effet, les produits pour les soins ou l'hygiène du corps ou des cheveux ont par exemple pour but d'optimiser la pénétration de produits actifs dans les couches superficielles de la peau. Par ailleurs, ces émulsions cosmétiques ont une composition bien particulière et sont caractérisées notamment par le fait que leur phase huileuse comprend une large variété de produits actifs tels que des huiles naturelles ou synthétiques, des hydrocarbures, des carbures halogénés, des esters d'acide minéral, des silicones, qui diffèrent selon l'application désirée. Contrairement à une composition cosmétique, une composition parfumante constitue un vecteur de parfum dont la fonction première est d'impartir une odeur à un produit. Sous forme d'émulsion, sa phase huileuse est essentiellement constituée d'ingrédients parfumants. Il est aisé de comprendre que les problèmes que l'on cherche à résoudre lors de la formulation d'émulsions tels que, en particulier, l'optimisation des paramètres susceptibles d'influencer la stabilité du produit, se posent de façon différente d'un domaine à l'autre. Les solutions à ces problèmes dépendent en effet de l'objectif recherché pour le produit, et plus précisément de la composition des phases continue et dispersée, et ne peuvent donc pas s'appliquer par simple analogie d'un domaine tel que la cosmétique ou la dermatologie, à la parfumerie. Les problèmes de stabilité spécifiques à la mise d'un parfum sous forme d'une émulsion sont d'ailleurs bien connus dans le domaine de la parfumerie (voir Cosmetics and Toiletries® magazine, Vol 109, pages 71-75, 1994) et ils sont liés à la nature des ingrédients émulsionnés, c'est à dire des ingrédients parfumants.

Par ailleurs, outre la stabilité chimique, une émulsion doit aussi répondre à certaines exigences en termes de stabilité physique. Par exemple, l'un des phénomènes typiquement associé à l'instabilité physique d'une émulsion est le mouvement ascendant et descendant des gouttes dispersées par rapport à la phase continue, nommé respectivement crémage ou sédimentation.

Les microémulsions sont des systèmes dispersés qui apportent une solution aux problèmes de déstabilisation menant au déphasage d'une émulsion classique. Ces systèmes sont largement décrits dans l'art antérieur, par exemple dans des demandes de brevets ou brevets tels que EP 516 508, EP 572 080, US 5,320,863, US 5,585,343 ou encore FR 2,703,926. Ces documents décrivent tous des dispersions d'eau et d'huile homogènes, transparentes et stables, ces propriétés provenant des larges quantités de tensioactifs et co-tensioactifs qui y sont ajoutées.

Les microémulsions et les émulsions constituent deux systèmes dispersés de nature bien distincte. En effet, alors que les émulsions sont des systèmes instables, les microémulsions sont stables et se forment de manière spontanée lorsque huile, eau, tensioactifs et co-tensioactifs sont mélangés ensemble. La stabilité thermodynamique d'une microémulsion se traduit notamment par le fait que la taille moyenne des gouttes du système ne varie pas avec le temps, contrairement au cas d'une émulsion. Les deux systèmes dispersés se différencient en outre par leurs propriétés optiques. En effet, les microémulsions ont une taille moyenne de gouttes bien inférieure à la longueur d'onde de la lumière et une distribution de taille de gouttes relativement étroite. Ainsi, ce type de formulation diffuse peu la lumière et est par conséquent transparent, alors que les émulsions ont une taille de gouttes comparable ou plus grande que la longueur d'onde de la lumière visible et une distribution de taille de gouttes plus large si bien qu'elles diffusent la lumière, donnant ainsi un effet optique allant du laiteux au translucide.

En tout état de cause, il est tout à fait clair aux yeux d'un homme du métier familier aux différents types de systèmes dispersés, que les émulsions et les microémulsions constituent deux systèmes dispersés de nature bien distincte.

Or, le but de la présente invention est notamment de réaliser des compositions n'incorporant pas de quantités importantes de tensioactifs par rapport à la quantité d'ingrédients parfumants, la présence de tensioactifs limitant considérablement la proportion de parfum qui peut être ajoutée au mélange. C'est pourquoi un système dispersé de type microémulsion ne convient pas pour la présente invention qui concerne donc un produit sous forme d'une émulsion huile-dans-l'eau et qui présente, en dépit de sa nature, une stabilité physique tout à fait surprenante.

Par ailleurs, un autre problème spécifique au domaine de la parfumerie est celui de la présence typique d'alcool.

En effet, dans la préparation de compositions parfumées telles que parfums, eaux de toilette, eaux de Cologne ou compositions déodorantes par exemple, l'utilisation d'éthanol en tant que solvant est très répandue. L'éthanol permet de bien solubiliser les ingrédients parfumants auxquels le parfumeur a recours. Il constitue ainsi le vecteur principal du parfumage corporel (parfums, eaux de toilette, déodorants, après-rasage etc.). Virtuellement sans odeur, c'est un très bon solvant qui s'évapore rapidement procurant ainsi une sensation de fraîcheur. Pour de telles raisons, la plupart des compositions parfumantes que l'on trouve sur le marché contiennent de l'éthanol, généralement entre 50 et 95% par volume.

Cependant, l'éthanol a une volatilité non négligeable, et il est parfois désirable d'obtenir des compositions parfumantes dont la teneur en alcool est très faible, ou même nulle.

Ainsi, on assiste actuellement sur le marché international à une tendance dirigée vers les parfums sans alcool, tendance née aussi bien des réglementations VOC (composés organiques volatiles) que de la préférence du consommateur pour des produits sans alcool adressés aux peaux sensibles et permettant une utilisation sans risque au soleil.

La demande de brevet JP 96225429 divulgue des compositions parfumantes dites "sans alcool" mais qui contiennent en général toujours une faible proportion d'alcool. Cette demande décrit des compositions parfumantes liquides ou sous forme de gels, comprenant en particulier des polymères solubles dans l'eau tels que des gommes. Ce type de polymère, bien connu dans le domaine des émulsions, est utilisé comme agent de stabilisation. Cependant, on sait également que l'utilisation de tels polymères a pour inconvénient de rendre les émulsions collantes au toucher. En outre, ces composants ont pour effet d'élever de façon considérable la viscosité des émulsions qui les contiennent. La demande de brevet japonaise citée précise d'ailleurs que les produits obtenus sont parfois visqueux, ou prennent même la forme de gels.

La présente invention se propose de résoudre le problème consistant à obtenir un produit dont la fonction est le parfumage, sous forme d'émulsion translucide sans alcool, susceptible d'être vaporisée, capable de contenir un pourcentage élevé de parfum, d'aspect et de toucher agréable, le tout allié à une bonne stabilité à long terme.

Aucun document de l'art antérieur ne décrit de telles émulsions et ne permet donc à ce jour de résoudre le problème posé.

### Exposé de l'Invention

La présente invention apporte une solution aux différents problèmes mentionnés, au moyen d'une composition parfumante sans alcool sous forme d'une émulsion vaporisable huile-dans-l'eau translucide contenant au moins un ingrédient parfumant, un système de tensioactif ayant une balance hydrophile-lipophile (HLB) supérieure ou égale à 10, et de l'eau. On entend ici par composition parfumante, une composition dont la fonction première consiste à modifier l'odeur d'un produit ou impartir une odeur à un produit ou une personne. La composition de l'invention a été optimisée de façon à pouvoir comprendre des quantités importantes de parfum, à savoir de 0,1 à 18% en poids, par rapport au poids total de l'émulsion. L'émulsion est également caractérisée par la variation de la taille moyenne de ses gouttes au cours du premier mois suivant sa formulation, cette variation étant comprise, à une température de 45°C, entre 0,1 et 30 nm.

L'émulsion, objet de la présente invention, présente de nombreux avantages pour l'utilisation que l'on désire en faire. Tout d'abord, comme mentionné plus haut, cette composition, destinée en particulier à être vaporisée sur la peau ou sur les cheveux, sur tout autre type de surface destinée à être parfumée, ou encore dans l'air ambiant, est particulièrement concentrée en ingrédients parfumants.

D'autre part, la taille moyenne des gouttes est telle que l'émulsion satisfait avantageusement aux critères de stabilité recherchés (absence d'effet de crémage ou de sédimentation) sans pour autant nécessiter la présence d'agents stabilisants tels que des polymères solubles dans l'eau, comme c'est le cas dans l'art antérieur. L'absence de tels composants présente l'avantage d'obtenir un produit de faible viscosité, susceptible d'être vaporisé, non collant au toucher. La viscosité des compositions selon l'invention est inférieure à 1 Pa.s.

En outre, la taille moyenne des gouttes de l'émulsion a un effet optique sur le produit final, dont l'aspect translucide avec des reflets bleutés est tout à fait apprécié par le consommateur et se trouve particulièrement adapté à une utilisation pour des produits dérivés d'un parfum, tels que des émulsions de parfum susceptibles d'être vaporisées. D'une façon générale, les propriétés optiques des compositions de l'invention sont caractérisées par une transmission, mesurée à une longueur d'onde de 600 nm, à 25°C, dans une cuve de 1 cm d'épaisseur, variant entre 10 et 90%.

D'autres avantages apparaîtront au cours de la description détaillée de l'invention et à travers les exemples d'illustration.

La phase dispersée de l'émulsion, objet de la présente invention, est essentiellement constituée d'ingrédients parfumants. Plus particulièrement, elle comprend de 50 à 99% de son poids d'ingrédients parfumants. Ces derniers sont présents en quantité variant entre 0,1 et 18% en poids par rapport au poids total de l'émulsion. De façon préférentielle, ils constituent entre 1 et 15% en poids par rapport au poids total de l'émulsion, voire entre 6 et 10% en poids par rapport au poids total de l'émulsion.

Les ingrédients parfumants pouvant être utilisés selon l'invention sont des ingrédients d'usage courant en parfumerie. Leur nature n'appelle pas une description plus détaillée ici, qui ne saurait d'ailleurs être exhaustive, l'homme de l'art étant à même de les choisir de part ses connaissances générales et en fonction de l'effet olfactif recherché. Ces ingrédients parfumants appartiennent à des classes chimiques aussi variées que les alcools, aldéhydes, cétones, esters, éthers, acétates, nitriles, hydrocarbures terpéniques, composés hétérocycliques azotés ou soufrés, ainsi que des huiles essentielles d'origine naturelle ou synthétique. Beaucoup de ces ingrédients sont d'ailleurs répertoriés dans des textes de référence tels que le livre de S. Arctander, Perfume and Flavor Chemicals, 1969, Montclair, New Jersey, USA, ou ses versions plus récentes, ou dans d'autres ouvrages de nature similaire, ainsi que dans la littérature scientifique et de brevets plus récente relative à l'art de la parfumerie.

La stabilité des compositions parfumantes de l'invention est tout à fait appréciable et adaptée aux durées de stockage typiques pour ce type de produits, grâce notamment à la présence d'un système de tensioactif dont la balance hydrophile-lipophile (HLB) est supérieure ou égale à 10, de préférence supérieure ou égale à 15. Par système de tensioactif, on entend ici un tensioactif seul ou un mélange de deux ou plusieurs agents de ce type. Le système de tensioactif selon l'invention se révèle particulièrement avantageux dans la mesure où il permet d'obtenir une émulsion stable pour une grande variété d'ingrédients parfumants utilisés dans la phase huileuse de l'émulsion. En effet, alors que les ingrédients parfumants présentent des caractéristiques très variées d'hydrophilicité et hydrophobicité, nous avons découvert que le système tensioactif de l'invention reste adapté pour une large variété d'ingrédients parfumants utilisés et permet d'obtenir une émulsion satisfaisant aux critères de stabilité nécessaires au stockage du produit.

Le système de tensioactif utilisé selon l'invention comprend des tensioactifs non-ioniques polyéthoxylés ou polypropoxylés. Le système de tensioactif utilisé selon l'invention est constitué de préférence par au moins un tensioactif non-ionique appartenant à l'une des familles constituées par les stéaryléthers de polyéthylèneglycol, les oleyléthers de polyéthylèneglycol (n), les nonylphényléthers de polyéthylèneglycol (n) et les polysorbates. D'autres alkyléthers de polyéthylèneglycol peuvent être utilisés selon l'invention. L'utilisation de mélanges de ces tensioactifs s'est avérée particulièrement avantageuse.

La valeur HLB du système doit être supérieure ou égale à 10. Selon un mode de réalisation de l'invention on utilise un système tensioactif constitué par un agent choisi dans la famille des éthers stéariques de polyéthylèneglycol (n). De façon préférée, on utilise un mélange de Steareth-20 et Steareth-21 dont la valeur HLB est de 15,3. De bons résultats ont aussi été obtenus en utilisant l'Oleth-20 (HLB = 15,3).

Le système de tensioactif selon l'invention est utilisé dans des proportions comprises entre 0,1 et 18 %, de préférence entre 1 et 10% en poids par rapport au poids total de l'émulsion. Les meilleurs résultats ont été obtenus en utilisant de 3 à 8% en poids d'agent tensioactif.

Comme il a été précisé plus haut, la phase huileuse de l'émulsion huile-dansl'eau de l'invention est constituée essentiellement d'ingrédients parfumants. D'autres substances peuvent encore être présentes dans la phase huileuse et ont donné de bons résultats. On peut citer notamment des paraffines lourdes telles que l'eicosane à 20 carbones, ou encore les fractions d'isoparaffine commercialisées sous le nom de Isopar^{®}, par exemple Isopar^{®} V, par la compagnie Exxon Chemicals, ou encore une autre fraction de paraffine, le Gemseal 60 commercialisé par Total. Ces substances sont utilisées comme agents stabilisateurs de l'émulsion.

La variation au cours du temps de la taille moyenne des gouttes de l'émulsion de l'invention constitue une caractéristique essentielle de la composition parfumante. En effet, cette variation bien caractéristique de l'émulsion en tant que système dispersé thermodynamiquement instable, est pourtant suffisamment faible pour rendre le produit tout à fait intéressant dans le sens où les phénomènes courants de déstabilisation tels que l'effet de crémage ou encore la sédimentation sont évités. En particulier, cette variation peut être mesurée par une méthode de diffusion dynamique de la lumière (décrite de façon détaillée dans l'Exemple 1) qui permet d'établir qu'au cours du premier mois suivant la formulation de l'émulsion selon l'invention la variation de la taille moyenne des gouttes du système, à une température de 45°C, est comprise dans une gamme allant de 0,1 à 30 nm.

Les compositions parfumantes selon l'invention peuvent être utilisées pour différents types d'applications de produits de parfumerie tels que des émulsions de parfum, d'eaux de toilette ou d'eaux de Cologne sans alcool, susceptibles d'être vaporisées sur la peau ou les cheveux. Elles peuvent également servir à parfumer d'autres types de surfaces telles que des tissus, du bois, du verre. Dans un autre mode de réalisation les compositions de l'invention peuvent même parfumer l'air ambiant et être ainsi utilisées comme diffuseurs de parfums.

La préparation des émulsions selon l'invention sera décrite de façon détaillée dans les exemples.

L'invention sera maintenant illustrée par les exemples suivants non limitatifs, dans lesquels les températures sont indiquées en degrés Celsius, les proportions des composés sont données en % en poids, et les abréviations ont le sens usuel dans l'art.

### Manières de Réaliser l'Invention

### Exemple 1

### Préparation d'une composition parfumante sans alcool sous forme d'émulsion huile-dans-l'eau translucide vaporisable

| Ingrédients | Parties en poids |
|---|---|
| Base parfumante * | 8,00 |
| Brij^{®} 98V ¹⁾ | 8,00 |
| Isopar^{®} V ²⁾ | 4,00 |
| Eau | 80.00 |
| Total | 100,00 |

| | |
|---|---|
| 1) Oleth-20 ; origine : Uniqema, Pays-Bas 2) origine : Exxon Chemicals, USA * La base parfumante a été obtenue par mélange des ingrédients suivants : | |

| Ingrédients | Parties en poids |
|---|---|
| Acétate de citronellyle | 3 |
| Acétate de géranyle | 9 |
| Acétate de linalyle | 276 |
| Aldéhyde C10 à 10% * | 3 |
| Aldéhyde C12 à 10% * | 12 |
| Anthranilate de méthyle | 16 |
| Essence de bergamote | 226 |
| Cetalox ^{® 1)} | 5 |
| Essence de citron | 318 |
| Dihydromyrcénol ²⁾ | 60 |
| Dipropylèneglycol | 20 |
| Elemi ³⁾ à 10% * | 20 |
| Lilial^{® 4)} | 3 |
| Ethyl linalol | 66 |
| 3-(4-Méthoxyphényl)-2-méthylpropanal ⁵⁾ à 10% * | 30 |
| Géraniol | 6 |
| Habanolide ^{® 6)} à 50% * | 130 |
| Hedione ^{® 7)} | 215 |
| Hedione ^{®} HC ⁸⁾ | 72 |
| Indol à 10% ** | 12 |
| Iso E super ⁹⁾ | 85 |
| Essence de lavandin grosso | 26 |
| Liffarome^{® 10)} à 1% * | 20 |
| Linalol | 40 |
| Essence de mandarine sfuma | 5 |
| Essence de menthe crépue à 10% * | 30 |
| Essence de Néroli bigarade | 130 |
| Essence d'orange Portugal Floride | 80 |
| Phénéthylol | 9 |
| Essence de petitgrain | 63 |
| Pipol | 5 |
| Essence de romarin | 16 |
| Terpinéol | 9 |
| Essence de violette | 50 |
| Zestover ¹¹⁾ à 1% * | 30 |
| Total | 2100 |

| | |
|---|---|
| * dans le dipropylèneglycol (DIPG) ** dans la triéthanolamine 1) 8,12-époxy-13,14,15,16-tétranorlabdane ; origine : Firmenich SA, Genève, Suisse 2) origine : International Flavors & Fragrances, USA 3) 5-allyl-1,2,3-triméthoxybenzène ; origine : Calchauvet, Grasse, France 4) 3-(4-tert-butylphényl)-2-méthylpropanal ; origine : Givaudan-Roure SA, Vernier, Suisse 5) origine : Firmenich SA, Genève, Suisse 6) pentadécénolide ; origine : Firmenich SA, Genève, Suisse 7) dihydrojasmonate de méthyle ; origine : Firmenich SA, Genève, Suisse 8) dihydrojasmonate de méthyle à haute teneur en isomère cis ; origine : Firmenich SA, Genève, Suisse 9) origine : International Flavors & Fragrances, USA 10) carbonate de 3-hexényle méthyle ; origine : International Flavors & Fragrances, USA 11) 2,4-diméthyl-3-cyclohexène-1-carbaldéhyde ; origine : Firmenich SA, Genève, Suisse | |

### Méthode de préparation de l'émulsion

Le système tensioactif rendu liquide et la phase huileuse ont été mélangés à température ambiante jusqu'à l'obtention d'un mélange homogène, avant d'ajouter la phase aqueuse. Le mélange a ensuite été chauffé de façon à obtenir une émulsion grossière, tout en le maintenant sous une faible agitation (200 tpm). Alors que la température augmentait, l'émulsion est devenue plus fine, puis plus visqueuse. Le chauffage a alors été stoppé et l'émulsion a été refroidie jusqu'à température ambiante (25°), toujours sous agitation.

On a ainsi obtenu une émulsion huile-dans-l'eau finement dispersée, d'aspect translucide (transmission de 80% à une longueur d'onde de 600 nm, une température de 25°, dans une cuve d'1 cm d'épaisseur) avec des reflets bleutés. La taille des particules a été mesurée à l'aide d'un granulomètre (Autosizer 4700, origine : Malvern ; angle de mesure : 90° ; longueur d'onde de laser : 532 nm ; température de mesure : 25° ; mode d'analyse monomodal en intensité). La taille moyenne des particules de la présente émulsion était de 29,5 nm après formulation. Après un mois à 45°, la taille moyenne des gouttes avait varié de 20 nm.

### Exemple 2

### Préparation d'une composition parfumante sans alcool sous forme d'émulsion huile-dans-l'eau translucide vaporisable

| Ingrédients | Parties en poids |
|---|---|
| Base parfumante * | 8,00 |
| Brij^{®} 98V ¹⁾ | 8,00 |
| Isopar^{®} V ²⁾ | 4,00 |
| Eau | 80,00 |
| Total | 100,00 |

| | |
|---|---|
| 1) voir Exemple 1 2) voir Exemple 1 * La base parfumante a été obtenue par mélange des ingrédients suivants : | |

| Ingrédients | Parties en poids |
|---|---|
| Acétate de benzyle | 250 |
| Acétate de pipol | 70 |
| Acétate de styrallyle | 230 |
| Aldéhyde phénylacétique | 10 |
| Ambrettolide ^{® 1)} | 10 |
| Astrotone | 300 |
| Essence de bergamote | 1160 |
| β-Ionone | 550 |
| Essence de cassis | 150 |
| Cetalox ^{® 2)} à 50% * | 60 |
| Essence de citron | 850 |
| Citronellol | 210 |
| Damascénone | 20 |
| 4-Décanolide | 20 |
| Dihydromyrcénol ³⁾ | 440 |
| Dipropylèneglycol | 20 |
| Ethyl linalol | 720 |
| 7-Méthyl-2H,4H-1,5-benzodioxépin-3-one ⁴⁾ | 100 |
| Floralozone ^{® 5)} | 50 |
| 3-(4-Méthoxyphényl)-2-méthylpropanal ⁴⁾ | 170 |
| Fructone ^{® 6)} | 100 |
| Galbex ^{® 4)} | 50 |
| γ-Damascone | 5 |
| Essence de géranium | 30 |
| Essence de pamplemousse | 100 |
| Habanolide ^{® 7)} | 1120 |
| Hedione ^{® 8)} | 2890 |
| Hedione ^{®} HC ⁹⁾ | 950 |
| Héliopropanal ¹⁰⁾ | 400 |
| Indol | 35 |
| Iso E Super ¹¹⁾ | 380 |
| Essence de lavandin grosso | 40 |
| Liffarome ^{®} 12) | 1 |
| Lilial ^{® 13)} | 1050 |
| Lyral ^{® 14)} | 430 |
| Essence de mandarine sfuma | 270 |
| Melonal ¹⁵⁾ | 3 |
| Essence de menthe crépue | 20 |
| Peony HS (HeadSpace) ⁴⁾ | 260 |
| Phénéthylol | 80 |
| Phénylhexanol | 50 |
| Pipol | 20 |
| Essence d'orange | 500 |
| Rosalva ¹⁶⁾ | 4 |
| Salicylate de benzyle | 400 |
| Salicylate de pipol | 400 |
| BHT à 10% ** | 200 |
| Zestover ¹⁷⁾ | 22 |
| Total | 15200 |

| | |
|---|---|
| * dans le 2-(2-éthoxyéthoxy)-1-éthanol ** dans le dipropylèneglycol 1) origine : Givaudan-Roure SA, Vernier, Suisse 2) 8,12-époxy-13,14,15,16-tétranorlabdane : origine : Firmenich SA, Genève, Suisse 3) origine : International Flavors & Fragrances, USA 4) origine : Firmenich SA, Genève, Suisse 5) 3-(4-éthylphényl)-2,2-diméthylpropanal + 3-(2-éthylphényl)-2,2-diméthylpropanal ; origine : International Flavors & Fragrances, USA 6) 2-méthyl-1,3-dioxalane-2-acétate d'éthyle ; origine : International Flavors & Fragrances, USA 7) pentadécénolide ; origine : Firmenich SA, Genève, Suisse 8) dihydrojasmonate de méthyle ; origine : Firmenich SA, Genève, Suisse 9) dihydrojasmonate de méthyle à haute teneur en isomère cis ; origine : Firmenich SA, Genève, Suisse 10) 3-(1,3-benzodioxol-5-yl)-2-méthylpropanal ; origine : Firmenich SA, Genève, Suisse 11) origine : International Flavors & Fragrances, USA 12) carbonate de 3-hexényle-méthyle ; origine : International Flavors & Fragrances, USA 13) 3-(4-tert-butylphényl)-2-méthylpropanal ; origine : Givaudan-Roure SA, Vernier, Suisse 14) 4-(4-hydroxy-4-méthylpentyl)-3-cyclohexène-1-carbaldéhyde + 3-(4-hydroxy-4-méthylpentyl)-3-cyclohexène-1-carbaldéhyde ; origine : International Flavors & Fragrances, USA 15) 2,6-diméthyl-5-heptanal ; origine : Givaudan-Roure SA, Vernier, Suisse 16) 9-décén-1-ol ; origine : International Flavors & Fragrances, USA 17) 2,4-diméthyl-3-cyclohexène-1-carbaldéhyde ; origine : Firmenich SA, Genève, Suisse | |

L'émulsion a été préparée de façon similaire à celle préparée dans l'Exemple 1.

On a obtenu une émulsion translucide (transmission de 69,4% à une longueur d'onde de 600 nm, une température de 25°, dans une cuve de 1 cm d'épaisseur), possédant une taille moyenne de particules de 30 nm après formulation. Après un mois à 45°, la taille moyenne des particules avait varié de 19 nm.

### Exemple 3

### Préparation d'une composition parfumante sans alcool sous forme d'émulsion huile-dans-l'eau translucide vaporisable

| Ingrédients | Parties en poids |
|---|---|
| Base parfumante * | 8,00 |
| Brij^{®} 78P ¹⁾ | 7,00 |
| Brij^{®} 721 ²⁾ | 1,00 |
| Gemseal 60 ³⁾ | 4,00 |
| Eau | 80.00 |
| Total | 100,00 |

| | |
|---|---|
| * voir Exemple 2 1) Steareth-20 ; origine : Uniqema, Pays-Bas 2) Steareth-21 ; origine : Uniqema, Pays-Bas 3) origine : Total, France | |

L'émulsion a été préparée de façon similaire à celle préparée dans l'Exemple 1.

La composition obtenue était translucide (transmission de 67,5% à 600 nm, à une température de 25°, dans une cuve de 1 cm d'épaisseur). La taille moyenne des particules était de 36,5 nm après la formulation et sa variation au bout d'un mois à 45°, de 1 nm.

### Exemple 4

### Préparation d'une composition parfumante sans alcool sous forme d'émulsion huile-dans-l'eau translucide vaporisable

| Ingrédients | Parties en poids |
|---|---|
| Base parfumante * | 8,00 |
| Brij^{®} 78P ¹⁾ | 6,00 |
| Brij^{®} 721 ²⁾ | 2,00 |
| Gemseal 60 ³⁾ | 4,00 |
| Eau | 80,00 |
| Total | 100,00 |

| | |
|---|---|
| * voir Exemple 2 1) Steareth-20 ; origine : Uniqema, Pays-Bas 2) Steareth-21 ; origine : Uniqema, Pays-Bas 3) origine : Total, France | |

L'émulsion a été préparée de façon similaire à celle préparée dans l'Exemple 1.

La composition obtenue était translucide (transmission de 74,4% à 600 nm, à une température de 25°, dans une cuve de 1 cm d'épaisseur). La taille moyenne des particules était de 37,5 nm après la formulation et sa variation au bout d'un mois à 45° de 2 nm.

### Exemple 5

### Préparation d'une composition parfumante sans alcool sous forme d'émulsion huile-dans-l'eau translucide vaporisable

| Ingrédients | Parties en poids |
|---|---|
| Base parfumante * | 8,00 |
| Brij^{®} 78P ¹⁾ | 4,00 |
| Brij^{®} 721 ²⁾ | 4,00 |
| Gemseal 60 ³⁾ | 4,00 |
| Eau | 80,00 |
| Total | 100,00 |

| | |
|---|---|
| * voir Exemple 2 1) Steareth-20 ; origine : Uniqema, Pays-Bas 2) Steareth-21 ; origine : Uniqema, Pays-Bas 3) origine : Total, France | |

L'émulsion a été préparée de façon similaire à celle préparée dans l'Exemple 1.

La composition obtenue était translucide (transmission de 45,7% à 600 nm, à une température de 25°, dans une cuve de 1 cm d'épaisseur). La taille moyenne des particules était de 46 nm après la formulation et sa variation au bout d'un mois à 45° de 0,5 nm.

## Revendications

1. Procédé de fabrication d'une composition parfumante sans alcool sous forme d'une émulsion vaporisable huile-dans-l'eau translucide comprenant les étape suivante :
a) mélanger un système de tensioactif rendu liquide et la phase huileuse constitué essentiellement d'ingrédients parfumants à température ambiante jusqu'à l'obtention d'un mélange homogène ;
b) ajouter au mélange obtenu à l'étape a) une phase aqueuse ;
c) chauffer le mélange obtenu à l'étape b) tout en maintenant une faible agitation pour former une émulsion de viscosité inférieure à 1 Pa.s. ;
d) stopper le chauffage et refroidir à température ambiante sous agitation.

2. Procédé selon la revendication 1, **caractérisé en ce que** le système de tensioactif possède une balance hydrophile-lipophile (HLB) supérieure ou égale à 10.

3. Procédé selon la revendication 1, **caractérisée en ce que** le système de tensioactif possède une balance hydrophile-lipophile supérieure ou égale à 15.

4. Procédé selon la revendication 1, **caractérisée en ce que** le système de tensioactif comprend au moins un tensioactif non-ionique polyéthoxylé ou polypropoxylé.

5. Procédé selon la revendication 4, **caractérisée en ce que** le tensioactif non-ionique polyéthoxylé appartient à l'une des familles constituées par les stéaryléthers de polyéthylèneglycol, les oleyléthers de polyéthylèneglycol, les nonylphényléthers de polyéthylèneglycol et les polysorbates.

6. Procédé selon la revendication 5, **caractérisée en ce que** le système de tensioactif comprend de l'Oleth-20.

7. Procédé selon revendication 5, **caractérisée en ce que** le système de tensioactif consiste en un mélange de Steareth-20 et Steareth-21.

8. Procédé selon la revendication 1, **caractérisée en ce que** le système de tensioactif représente de 0,1 à 18% en poids du poids total de la composition.

9. Procédé selon la revendication 1, **caractérisée en ce que** le système de tensioactif représente 3 à 8% en poids du poids total de la composition.

10. Procédé selon la revendication 1, **caractérisée en ce que** la proportion d'ingrédient parfumant est comprise entre 0.1 et 18% en poids par rapport au poids total de la composition.

11. Procédé selon la revendication 1, **caractérisée en ce que** la proportion d'ingrédient parfumant est comprise entre 1 et 15% en poids par rapport au poids total de la composition.

12. Procédé selon la revendication 1, **caractérisée en ce que** la proportion d'ingrédient parfumant est comprise entre 6 et 10% en poids par rapport au poids total de la composition.

13. Procédé selon la revendication 1, **caractérisée en ce que** la phase huileuse de l'émulsion comprend de 50 à 99% de son poids d'ingrédient parfumant.

14. Procédé selon la revendication 1, caractérisée en ce la transmission de la composition mesurée, à une longueur d'onde de 600 nm, à une température de 25°C, dans une cuve d'une épaisseur de 1 cm, varie entre 10 et 90%.
